# EUROPEAN PATENT APPLICATION

(11) **EP 1 541 105 A1**
(43) Date of publication of application: **15.06.2005**
(21) Application number: 03795267.8
(22) Date of filing: 29.08.2003
(51) Int. Cl.: A61F 13/514

(54) **DISPOSABLE WEARING ARTICLE**

(30) Priority: 30.08.2002 JP 2002255673; 22.08.2003 JP 2003208493
(71) Applicant: UNI-CHARM CO., LTD., Kawanoe-shi Ehime 799-0111 (JP)
(72) Inventor: SASAKI, Toru, Technical Center UNI-CHARM CO. LTD., Mitoyo-gun, Kagawa 769-1602 (JP); ITO, Kyoko, c/o Technical Center UNI-CHARM CO. LTD, Mitoyo-gun, Kagawa 769-1602 (JP)
(74) Representative: Sperling, Rüdiger, Dipl.-Ing.
(86) International application number: PCT/JP2003/011091
(87) International publication number: WO 2004/024047

(57) **Abstract**

Here is disclosed a disposable wearing article in which a content of finishing textile oil in fibrous nonwoven fabric 42 constituting a backsheet 12 is 0.04wt% or less based on a weight of component fibers of the nonwoven fabric 42. In a front waist region of the wearing article, transversely opposite lateral portions 20 are provided with a pair of tape tabs 26 extending in a transverse direction. Each of these tape tabs 26 comprises a fixed section 27 permanently bonded to the outer surface of the backsheet 12 by means of adhesive 30, a first free subsection 28 temporarily bonded to the outer surface of the backsheet 12 by means of adhesive 31 applied on the inner surface of the first free subsection 28 and second free subsection 29 temporarily bonded to the outer surface of the first free subsection 28 by means of adhesive 32 applied to the inner surface of the second free subsection 29.

## Description

### TECHNICAL FIELD OF THE INVENTION

The present invention relates to disposable wearing articles such as an infant diaper, incontinence diaper, training pants, diaper cover and the like all adapted to absorb bodily discharge and to retain these bodily discharges therein.

### RELATED ART

As an example of such disposable wearing article, a pants-type disposable diaper is well known, which is configured by front and rear waist regions opposed to each other and a crotch region extending between these waist regions and provided, in the crotch region, on the outer surface of a backsheet with tape tabs for disposal adapted to hold the used diaper contaminated with bodily discharges in a predetermined shape (See Japanese Patent Publication No. 1996-84746A).

The diaper disclosed in the above cited Application comprises a liquid-pervious topsheet facing the wearer's skin, a liquid-impervious backsheet facing away from the wearer's skin and a liquid-absorbent core interposed between these sheets. In this diaper, the front and rear waist regions are connected to each other along transversely opposite lateral portions thereof by means of a plurality of heat-sealing lines arranged intermittently in a longitudinal direction of the diaper so as to define a waist-hole and a pair of leg-holes. Each of the tape tabs for the above described purpose comprises a fixed tape section firmly bonded to the outer surface of the backsheet by means of adhesive and a free tape section being contiguous to the fixed tape section and placed upon the outer surface of the fixed tape section. The free tape section is coated on its inner surface with self-adhesive by means of which the free tape section is temporarily bonded to the outer surface of the fixed tape section. Plastic film is used as stock material for this tape tab for disposal of the used diaper.

To dispose of the diaper contaminated with bodily discharges, the front and rear waist regions are folded in two, respectively, these waist regions placed upon the crotch region, then the free tape section is peeled off from the fixed tape section and anchored on the outer surface of the backsheet by means of self-adhesive. In this way, the diaper is retained by the tape tabs in its folded state and ready for disposal.

### DISCLOSURE OF THE INVENTION

For the backsheet formed by fibrous nonwoven fabric, it is well known to coat the nonwoven fabric with finishing textile oil or to mix such oil into the component fibers of the nonwoven fabric to make the component fibers of the nonwoven fabric flexible and smooth and to provide the nonwoven fabric with water-repellency. The finishing textile oil may be selected from the group consisting of anionic, nonionic, cationic and ampholytic surfactants and a mixture thereof. In the case of anionic or nonionic surfactant, the surfactant is sometimes mixed with any one of fat and oil, wax, fatty acid, higher alcohol, mineral oil and hardened oil in order to facilitate such surfactant to adhere to the fibers.

Assumed that the backsheet of the diaper disclosed by the above-cited Application is formed by fibrous nonwoven fabric, and this nonwoven fabric contains the finishing textile oil, there is possibility, depending on compatibility between the finishing textile oil and adhesive, that the finishing textile oil might progressively deteriorate an adhesive force of the adhesive and an adhesive strength of the tape tabs to the backsheet might be deteriorated as time elapses. Deterioration of the adhesive strength of the fixed tape section to the backsheet would lead to a serious inconvenience that the fixed tape sections of the tape tabs are apt to be peeled off from the outer surface of the backsheet and the fixed tape sections may sometimes be unintentionally peeled off from the backsheet as the free tape sections are peeled off from the associated fixed tape sections for disposal of the diaper.

It is an object of the present invention to provide a disposable wearing article improved so that a desired adhesive strength of tape strips to the outer surface of the backsheet can be maintained even after a relatively long period has elapsed.

According to the present invention, there is provided a disposable wearing article having front and rear waist regions opposed to each other, a crotch region extending between these waist regions, and tape tabs attached to the outer surface of the backsheet facing away from a wearer's skin.

The present invention further comprises the backsheet being formed by a fibrous nonwoven fabric and a content of finishing textile oil in the nonwoven fabric is 0.04wt% or less (as measured in accordance with the solvent extraction process specified by JIS L 1015 7.22) on the basis of a weight of fibers constituting the nonwoven fabric and the tape tabs being permanently bonded to the outer surface of the backsheet by means of adhesive.

The present invention includes the following embodiments:
(1) Each of the tape tabs comprises a fixed section permanently bonded to the outer surface of the backsheet by means of the adhesive and a first free subsection being contiguous to the fixed section so as to be placed upon the outer surface of the fixed section and temporarily bonded to the outer surface of the fixed section by fastening means formed on the inner surface of the first free subsection.
(2) The adhesive applied on the fixed section of the tape tab is pressure-sensitive adhesive.
(3) Each of the tape tabs further comprises a second free subsection provided on transversely opposite side edge portions of the front or rear waist region so as to extend laterally outward therefrom, respectively, and being contiguous to the first free subsection so as to be placed upon the outer surface of the first free subsection and the second free subsection is temporarily bonded to the outer surface of the first free subsection by fastening means formed on the inner surface of the second free subsection.
(4) The tape tabs are provided on transversely opposite side edge portions of the front or rear waist region so as to extend longitudinally and means to connect the front and rear waist regions to each other are formed on the outer surfaces of the tape tabs.
(5) Adhesive strength of the fixed tape section to the backsheet is 4N/20mm or higher as measured after the wearing article has been left at a temperature of 50°C and at a humidity of 60% for a week.
(6) The backsheet has elastic stretchability in its transverse direction and the tape tabs are bonded to said backsheet while the backsheet is stretched in its transverse direction.
(7) A plurality of elastic members spaced one from another by a given dimension are contractibly attached to the backsheet and the elastic stretchability of the backsheet in its transverse direction is given by the elastic members.
(8) The fibrous nonwoven fabric constituting the backsheet is made of spun bond nonwoven fabric or spun lace nonwoven fabric.
(9) The fastening means are provided in the form of pressure-sensitive adhesive or hooks constituting so-called mechanical fastener.

### BRIEF DESCRIPTION OF THE DRAWINGS

Fig. 1 is a perspective view showing a diaper according to a typical embodiment of the present invention as partially broken away;
Fig. 2 is a sectional view taken along a line 2-2 in Fig. 1;
Fig. 3 is a perspective view showing the used diaper rolled up for disposal;
Fig. 4 is a perspective view showing a diaper according to another embodiment of the present invention;
Fig. 5 is a sectional view taken along a line 5-5 in Fig. 4;
Fig. 6 is a perspective view showing a diaper according to still another embodiment of the present invention;
Fig. 7 is a sectional view taken along a line 7-7 in Fig. 6;
Fig. 8 is a plan view showing a diaper according to further another embodiment of the present invention;
Fig. 9 is a sectional view taken along a line 9-9 in Fig. 8; and
Fig. 10 is a perspective view showing the diaper in which the lateral portions of the front waist region are connected with the lateral portions of the rear waist region.

### DESCRIPTION OF THE BEST MODE FOR WORKING OF THE INVENTION

Details of a disposable wearing article according to the present invention will be more fully understood from the description given hereunder in reference with the accompanying drawings.

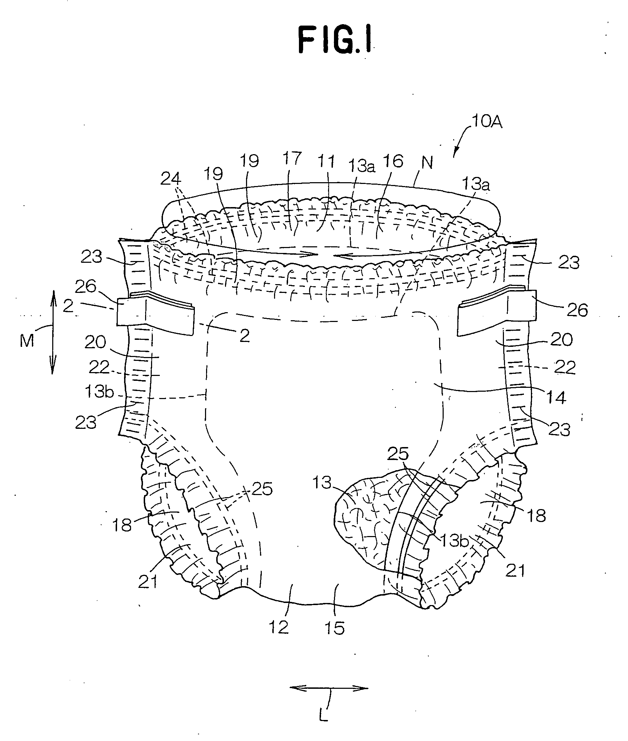

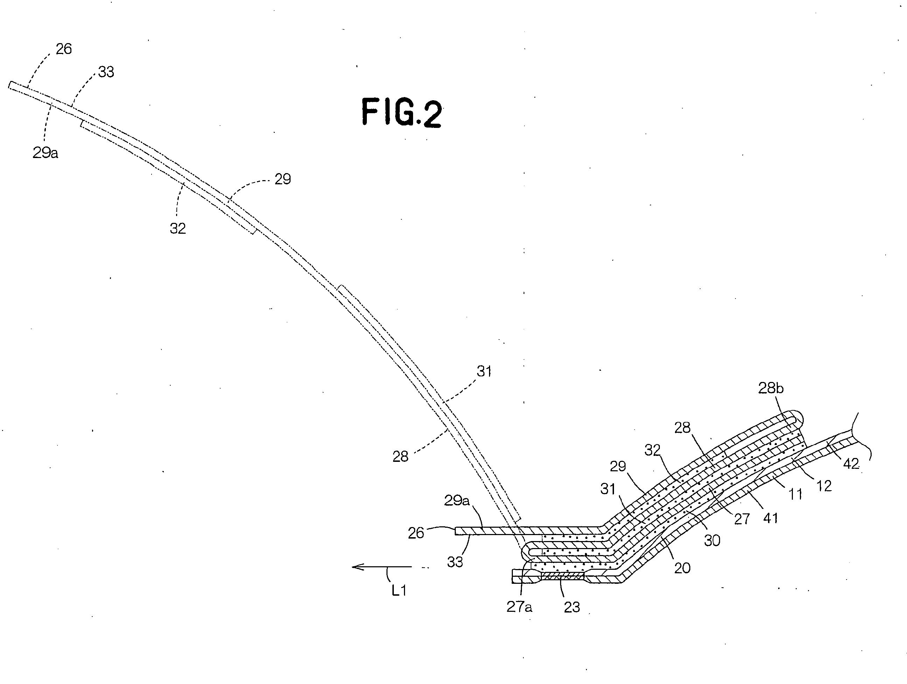

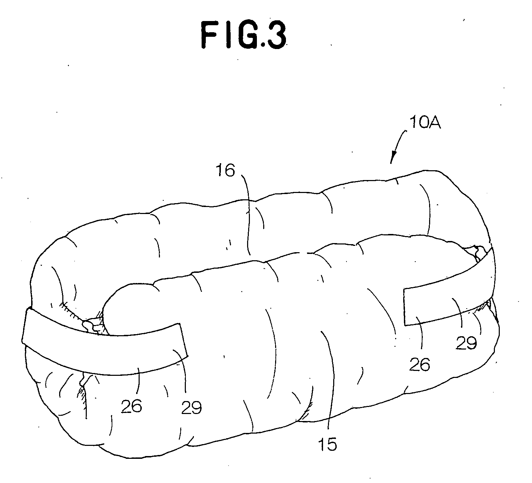

Fig. 1 is a perspective view showing a diaper 10A according to a typical embodiment of the present invention as partially broken away, Fig. 2 is a sectional view taken along a line 2-2 in Fig. 1 and Fig. 3 is a perspective view showing the used diaper 10A rolled up for disposal. In Fig. 1, a transverse direction is indicated by an arrow L and a longitudinal direction is indicated by an arrow M. In Fig. 2, first and second free subsections 28, 29 developed outward in the transverse direction of the diaper 10A as indicated by an arrow L1 are indicated by chain double-dashed line. Expression used herein "inner surfaces" of top- and backsheets 11, 12 and a tape tab 26 should be understood to mean the respective surfaces thereof facing the core 13 and expression used herein "outer surfaces" of these sheets and tape tab should be understood to mean the respective surfaces thereof facing away from the core 13.

The diaper 10A comprises the liquid-pervious topsheet 11 facing the wearer's skin, the liquid-impervious backsheet 12 facing away from the wearer' s skin and the liquid-absorbent core 13 interposed between the top- and backsheets 11, 12. The topsheet 11 is formed by hydrophilic fibrous nonwoven fabric and the backsheet 12 is formed by hydrophobic fibrous nonwoven fabric. The core 13 is intermittently bonded to the inner surface of at least one of these sheets 11, 12 by means of adhesive (not shown).

The diaper 10A is configured in a pants-type having a waist-hole 17 and a pair of leg-holes 18 and has front and rear waist regions 14, 16 opposed to each other and a crotch region 15 extending between these waist regions 14, 16. The diaper 10A further comprises waist-surrounding end portions 19 lying outside longitudinally opposite ends 13a of the core 13 and extending in the transverse direction and lateral portions 20, 21, 22 lying outside transversely opposite side edges 13b of the core 13 and extending in the longitudinal direction.

Between the waist-opening 17 and a pair of the leg-holes 17, the lateral portions 20, 22 of the front and rear waist regions 14, 16 are put flat and bonded together by means of a plurality of heat-sealing lines 23 arranged intermittently in the longitudinal direction in the vicinity of the respective marginal edge zones of the lateral portions 20, 22. The opposite lateral portions 21 of the crotch region 15 describe circular arcs which are convex inward in the transverse direction so that the crotch region 15 has a width narrower than those of the front and rear waist regions 14, 16. Accordingly, the diaper 10A presents a substantially hourglass-like planar shape as the front and rear waist regions 14, 16 are disconnected from each other and the diaper 10A is developed in the longitudinal direction. Along the waist-surrounding end portions 19 and the lateral portions 20, 21, 22, the top- and backsheets 11, 12 are placed upon each other and have respective inner surfaces intermittently bonded together by means of adhesive (not shown).

The waist-surrounding end portions 19 are provided with a plurality of waist-surrounding elastic members 24 extending in the transverse direction and the lateral portions 21 of the crotch region 15 are provided with a plurality of leg-surrounding elastic members 25 so that these elastic members 24, 25 may be contractible. The waist-surrounding elastic members 24 and the leg-surrounding elastic members 25 are interposed between the top- and backsheets 11, 12 and intermittently bonded to the inner surfaces of these sheets 11, 12 by means of adhesive (not shown).

In the front waist region 14, a pair of tape tabs 26 are attached to the respective lateral portions 20 and extend in the transverse direction. Each of these tape tabs 26 is folded so as to present a Z-shaped cross-section and adapted to be unfolded from the associated lateral portion 20 outward in the transverse direction as indicated by double chain-dashed line in Fig. 2. The tape tab 26 has a fixed section 27 extending in the transverse direction, a first free tape subsection 28 being contiguous to the fixed section 27 and extending also in the transverse direction and a second free subsection 29 being contiguous to the first free subsection 28 and extending in the transverse direction.

The fixed section 27 has its inner surface permanently bonded to the outer surface of the backsheet 12 by means of pressure-sensitive adhesive 30. The first free subsection 28 is folded along an outer end 27a of the fixed section 27 toward the outer surface of the fixed section 27. The first free subsection 28 is placed upon the outer surface of the fixed section 27 and temporarily bonded to the outer surface of the fixed section 27 by means of pressure-sensitive adhesive 31 serving as fastening means applied on the inner surface of the first free subsection 28. The second free subsection 29 is folded along an inner end portion 28b of the first free subsection 28 toward the outer surface of the first free subsection 28. The second free subsection 29 is placed upon the outer surface of the first free subsection 28 and temporarily bonded to the outer surface of the first free subsection 28 by means of pressure-sensitive adhesive 32 serving as fastening means applied on the inner surface of the second free subsection 29. The second free tape subsection 22 is formed in the vicinity of its outer end portion 29a with a finger grip 33 coated with no pressure-sensitive adhesive.

The pressure-sensitive adhesive 31 is applied on the entire inner surface of the first free subsection 28 and the pressure-sensitive adhesive 32 is applied on the entire inner surface of the second free subsection 29 except the finger grip 33 (zone in the vicinity of the outer end portion 29a). The entire outer surface of the fixed section 27 and the entire outer surface of the first free subsection 28 are coated with release agent (not shown). Expression "inner surfaces" of the first and second free subsections 28, 29 used herein refers to the surfaces facing the core 13 when these free subsections 28, 29 are folded and expression "outer surfaces" of these free subsections 28, 29 used herein refers to the surfaces facing away from the core 13 when these subsections 28, 29 are folded.

The pressure-sensitive adhesive 30, 31 may be selected from a group consisting of hot melt adhesive, acrylic adhesive and rubber adhesive. The release agent may be silicone resin, fluorocarbon resin, amino alkyd resin or polyester resin, all being well known for those skilled in the art.

A peel force required to develop the tape tabs 26 against the adhesive effect of the pressure-sensitive adhesive 31 is preferably lower than a peel force against the adhesive effect of the pressure-sensitive adhesive 32 in order to facilitate the tape tabs 26 to be developed. Alternatively, the pressure-sensitive adhesive 30 and the pressure-sensitive adhesive 32 may of the same type so far as the pressure-sensitive adhesive 32 exhibits an adhesive force lower than the pressure-sensitive adhesive 30 to the backsheet 12. This is for the reason that a period for which the pressure-sensitive adhesive 32 is retained on the backsheet is shorter than a period for which the pressure-sensitive adhesive 30 is retained on the backsheet and the adhesive force of the pressure-sensitive adhesive 32 is correspondingly less affected by the presence of the finishing textile oil in the nonwoven fabric.

The tape tabs 26 are used for disposal of the used diaper 10A contaminated with bodily discharges. Disposal of the diaper 10A may be carried out in accordance with a procedure as will be described. After the diaper 10A has been taken off from the wearer' s body, the crotch region 15 is rolled up toward the front waist region 14 in the longitudinal direction, the finger grips 33 of the respective second free subsections 29 are held between the fingers, and then the second free subsections 29 are pulled inward in the transverse direction. By pulling the second free subsections 29 inward in the transverse direction, the second free subsections 29 are peeled off from the outer surface of the associated first free subsections 29, then the first free subsections 28 also are peeled off from the outer surface of the fixed section 27. In this manner, the first and second free subsections 28, 29 are developed outward in the transverse direction as indicated by the arrow L1. Now the inner surfaces of the second free subsections 29 are fastened to the outer surface of the crotch region 15 (the outer surface of the backsheet 12) by means of the pressure-sensitive adhesive 32. As shown by Fig. 3, the diaper 10A is maintained in the rolled up state and can be disposed in this rolled up state.

The tape tabs 26 are useful not only for disposal of the used diaper 10A but also for adjustment of the waist-circumferential dimension N as the diaper 10A is put on the wearer's body. To adjust the waist-circumferential dimension N of the diaper 10A, while not illustrated, the first and second free subsections 28, 29 of the tape tabs 26 may be developed outward in the transverse dimension, then the tape tabs 26 may be pulled toward the transversely middle point and the second free subsections 29 of the tape tabs 26 may be anchored on the outer surface of the rear waist region 16 (the outer surface of the backsheet 12). Upon anchoring of the free subsections 29 on the rear waist region 16, each of the lateral portions 22 extending between the fixed section 28 and the free subsections 28, 29 has its transverse dimension reduced. In this way, the waist-circumferential dimension N of the diaper 10A can be reduced.

In the diaper 10A, a content of finishing textile oil in the fibrous nonwoven fabric 42 forming the backsheet 12 is 0.04wt% or less based on a weight of fiber constituting the nonwoven fabric 42 and an adhesive strength of the fixed section 27 of the tape tab 26 to the backsheet 12 is 4N/20mm or higher as measured after the diaper 10A has been left at a temperature of 50°C and at a humidity of 60% for a week. The content of finishing textile oil in nonwoven fabric 42 was measured in accordance with solvent extraction process specified by JIS L 1015 7.22. The adhesive strength of the fixed section 27 to the backsheet 12 was measured by a method as follows:
(1) The diaper 10A is left at a temperature of 50°C and at a humidity of 60% for a week without any load applied thereon and thereafter left at a temperature of 20°C and at a humidity of 20% further for 24 hours or longer.
(2) The fixed section 27 is cut off together with the backsheet 12 from the diaper 10A to obtain a sample piece for measurement.
(3) Adhesive strength was measured using INSTRON 5543 manufactured by INSTRON JAPAN CO. LTD under measurement condition as follows: chuck-distance: 40mm; peeling rate: 300mm/min; peeling angle: 180°; atmosphere: a temperature of 20°C and a humidity of 60%; measuring range: a range defined between a point 10mm behind a starting point of peeling the fixed section 27 of the tape tab 26 off from the backsheet 12 and a point 30mm before completion of this peeling. An average adhesive strength in this range was calculated.

The finishing textile oil is used to make the component fibers of the nonwoven fabric 42 flexible and smooth and to provide the nonwoven fabric 42 with water-repellency. The finishing textile oil may be applied either on the nonwoven fabric 42 or mixed into the component fibers of the nonwoven fabric 42. The finishing textile oil may be selected from a group consisting of anionic, nonionic, cationic and ampholytic surfactants and a mixture thereof. In the case of anionic or nonionic surfactant, the surfactant may be mixed with any one of fat and oil, wax, fatty acid, higher alcohol, mineral oil and hardened oil in order to facilitate such surfactant to adhere to the fibers.

If the content of the finishing textile oil exceeds 0.04wt%, the finishing textile oil may progressively deteriorate the adhesive force of adhesive 30 as time elapses until the adhesive strength of the fixed section 27 may fall below 4N/20mm. If the adhesive strength of the fixed section 27 is less than 4N/20mm, the tape tabs 26 will be readily peeled off from the outer surface of the backsheet 12. In a consequence, the fixed section 27 may sometimes unintentionally be peeled off from the outer surface of the backsheet 12 as the tape tabs 26 are pulled in the transverse direction.

If the content of the finishing textile oil for the nonwoven fabric 42 is less than 0.04wt% based on the weight of the component fibers constituting the nonwoven fabric 42, deterioration of the adhesive strength of the adhesive 30 can be avoided and the desired adhesive strength of the fixed section 27 of the tape tab 26 to the backsheet 12 can be maintained even after the diaper 10A has been left for a long period. The fixed section 27 of the tape tab 26 can be held on the backsheet 12 at the adhesive strength of 4N/20mm or higher even after the diaper 10A has been left at a temperature of 50°C and a humidity of 60% for a week, so there is no apprehension that the tape tab 26 might be unintentionally peeled off from the outer surface of the backsheet 12 even when the tape tabs 26 are pulled in the transverse direction to develop the first and second free subsections 28, 29 even after the diaper 10A has been left for a long period.

In this diaper 10A, the content of the finishing textile oil in the fibrous nonwoven fabric 41 forming the topsheet 11 also is preferably 0.04wt% or less based on the weight of the component fibers constituting the nonwoven fabric 41 and the adhesive strength between the top- and backsheets 11, 12 is preferably 0.15N/25mm or higher. The content of the finishing textile oil in the nonwoven fabric 41 is measured by the same method as for the backsheet 12. The adhesive strength between the top- and backsheets 11, 12 is measured by the same method as for the adhesive strength of the tape tab 26 to the backsheet 12 (It should be understood that the sheet sample for measurement comprises the top- and backsheets 11, 12 bonded to each other).

If the content of the finishing textile oil in the fibrous nonwoven fabric 41 exceeds 0.04wt%, the finishing textile oil may progressively deteriorate the adhesive force of adhesive 30 as time elapses until the adhesive strength between the top- and backsheets 11, 12 may fall below 0.15N/20mm. Assumed that the adhesive strength between the top- and backsheets 11, 12 is less than 0.15N/25mm, these sheets 11, 12 may sometimes be peeled off from each other as the tape tabs 26 are pulled in the transverse direction and, in consequence, the force pulling the tape tabs 26 may be exerted upon the backsheet 12 alone, leading to breakage of the backsheet 12. So far as the adhesive strength between the top- and backsheets 11, 12 is 0.15N/25mm or higher, there is no anxiety that the top- and backsheets 11, 12 might be peeled off from each other in the vicinity of regions in which the tape tabs 26 are attached to the diaper 10A even when the tape tabs 26 are pulled in the transverse direction. Furthermore, the tape tabs 26 will be easily developed outward in the transverse direction because the force pulling the tape tabs 26 is evenly exerted upon the top- and backsheets 11, 12.

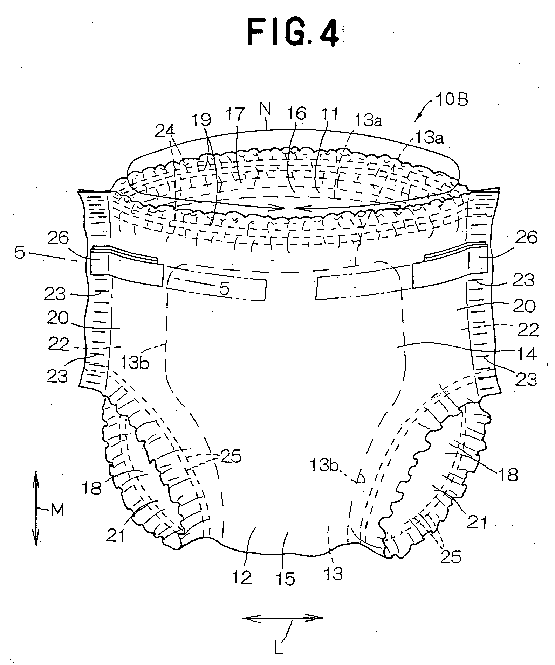

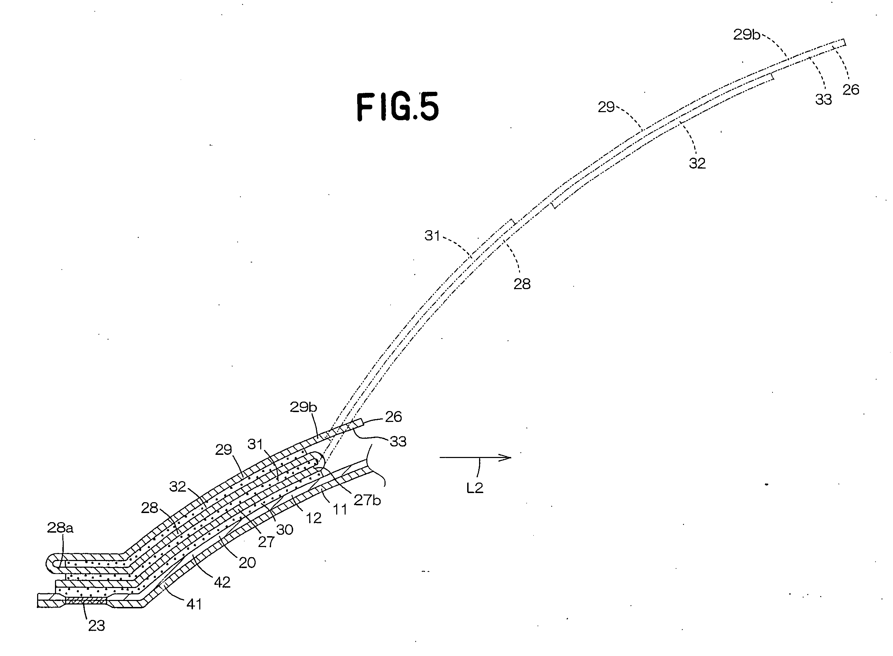

Fig. 4 is a perspective view showing a diaper 10B according to another embodiment of the present invention as partially broken away, Fig. 5 is a sectional view taken along a line 5-5 in Fig. 4. In Fig. 4 also, a transverse direction is indicated by the arrow L and a longitudinal direction is indicated by the arrow M. In Fig. 4, the first and second free subsections 28, 29 developed inward in the transverse direction of the diaper 10B as indicated by an arrow L2 are indicated by chain double-dashed line.

This diaper 10B is similar to the diaper 10A except that the first and second free subsections 28, 29 of the respective tape tabs 26 extend inward in the transverse direction of the diaper 10B as these first and second free subsections 28, 29 are developed. The parts similar to those in the diaper 10A will be designated by the similar reference numerals and detailed description thereof will not be repeated.

In the front waist region 14, the tape tabs 26 are attached to the respective lateral portions 20 and extend in the transverse direction. Each of these tape tabs 26 is folded so as to present a Z-shaped cross-section and adapted to be unfolded from the associated lateral portion 20 inward in the transverse direction as indicated by double chain-dashed line in Fig. 5. The tape tab 26 has the fixed section 27 extending in the transverse direction, the first free tape subsection 28 being contiguous to the fixed section 27 and extending also in the transverse direction and the second free subsection 29 being contiguous to the first free subsection 28 and extending in the transverse direction.

The fixed section 27 has its inner surface bonded to the outer surface of the backsheet 12 by means of pressure-sensitive adhesive 30. The first free subsection 28 is folded along an inner end 27b of the fixed section 27 toward the outer surface of the fixed section 27. The first free subsection 28 is placed upon the outer surface of the fixed section 27 and temporarily bonded to the outer surface of the fixed section 27 by means of pressure-sensitive adhesive 31 serving as fastening means applied on the inner surface of the first free subsection 28. The second free subsection 29 is folded along an inner end portion 28a of the first free subsection 28 toward the outer surface of the first free subsection 28. The second free subsection 29 is placed upon the outer surface of the first free subsection 28 and temporarily bonded to the outer surface of the first free subsection 28 by means of pressure-sensitive adhesive 32 serving as fastening means applied on the inner surface of the second free subsection 29. The second free tape subsection 22 is formed in the vicinity of its inner end portion 29b with the finger grip 33 coated with no pressure-sensitive adhesive. The outer surface of the fixed section 27 and the outer surface of the first free subsection 28 are entirely coated with release agent (not shown).

The tape tabs 26 are useful for disposal of the diaper 10B contaminated with bodily discharges. Disposal of the diaper 1B may be carried out in accordance with a procedure as will be described. After the diaper 1B has been taken off from the wearer's body, the crotch region 15 is rolled up toward the front waist region 14 in the longitudinal direction, the finger grips 33 of the respective second free subsections 29 are held between the fingers, and then the second free subsections 29 are pulled outward in the transverse direction. By pulling the second free subsections 29 outward in the transverse direction, the second free subsections 29 are peeled off from the outer surface of the associated first free subsections 29, then the first free subsections 28 also are peeled off from the outer surface of the fixed section 27. In this manner, the first and second free subsections 28, 29 are developed inward in the transverse direction. Now the inner surfaces of the second free subsections 29 are fastened to the outer surface of the crotch region 15 (the outer surface of the backsheet 12) by means of the pressure-sensitive adhesive 32 as indicated by chain double-dashed lines in Fig. 5. Similarly to the case shown by Fig. 1, the used diaper 10B is held by the tape tabs 26 in the rolled up state and can be disposed in this state.

The tape tabs 26 are useful also for adjustment of the waist-circumferential dimension as the diaper 10B is put on the wearer's body. To adjust the waist-circumferential dimension N of the diaper 10B, the first and second free subsections 28, 29 of the tape tabs 26 may be developed inward in the transverse dimension, then the tape tabs 26 may be pulled toward the transversely middle point and the second free subsections 29 of the tape tabs 26 may be anchored on the outer surface of the front waist region 14 (the outer surface of the backsheet 12) by means of the adhesive 32. Upon anchoring of the free subsections 29 on the front waist region 14, each of the lateral portions 22 extending between the fixed section 28 and the free subsections 28, 29 has its transverse dimension is reduced. In this way, each of the lateral portions 22 extending between the fixed section 27 and the free subsections 28, 29 has its transverse dimension reduced. In this way, the waist-circumferential dimension N of the diaper 10B can be reduced.

In the diaper 10B, a content of finishing textile oil in the fibrous nonwoven fabric 42 forming the backsheet 12 is 0.04wt% or less based on a weight of fiber constituting the nonwoven fabric 42 and an adhesive strength of the fixed section 27 of the tape tab 26 to the backsheet 12 is 4N/20mm or higher as measured after the diaper 10A has been left at a temperature of 50°C and at a humidity of 60% for a week. The content of finishing textile oil in nonwoven fabric 42 was measured in accordance with solvent extraction process specified by JIS L 1015 7.22. The adhesive strength of the fixed section 27 to the backsheet 12 was measured by the method same as the method having been described in connection with Fig. 1. The pressure-sensitive adhesive 30, 31, 32, the release agent and the finishing textile oil are same as those used in the embodiment shown by Fig. 1.

If the content of the finishing textile oil for the nonwoven fabric 42 is less than 0.04wt% based on the weight of the component fibers constituting the nonwoven fabric 42, deterioration of the adhesive strength of the adhesive 30 can be avoided and the desired adhesive strength of the fixed section 27 of the tape tab 26 to the backsheet 12 can be maintained even after the diaper 10B has been left for a long period. The fixed section 27 of the tape tab 26 can be held on the backsheet 12 at the adhesive strength of 4N/20mm or higher even after the diaper 10B has been left at a temperature of 50°C and a humidity of 60% for a week, so there is no apprehension that the tape tab 26 might be unintentionally peeled off from the outer surface of the backsheet 12 even when the tape tabs 26 are pulled in the transverse direction to develop the first and second free subsections 28, 29 even after the diaper 10B has been left for a long period.

In this diaper 10B, the content of the finishing textile oil in the fibrous nonwoven fabric 41 forming the topsheet 11 also is preferably 0.04wt% or less based on the weight of the component fibers constituting the nonwoven fabric 41 and the adhesive strength between the top- and backsheets 11, 12 is preferably 0.15N/25mm or higher.

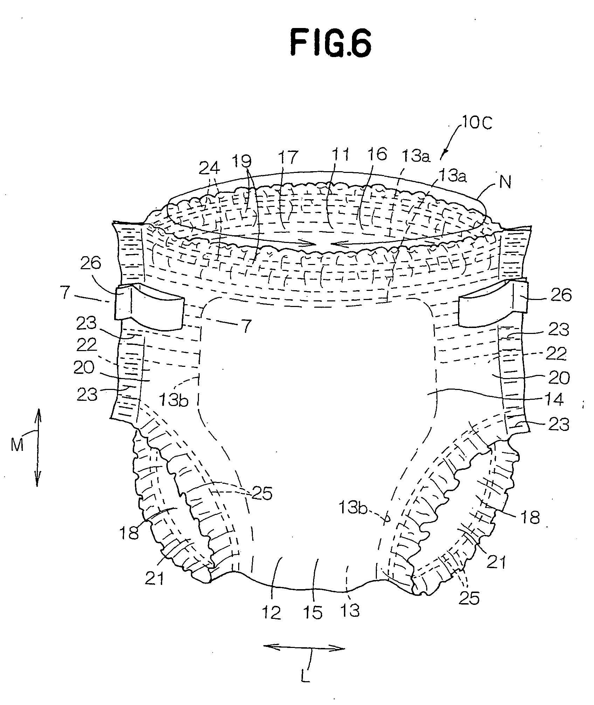

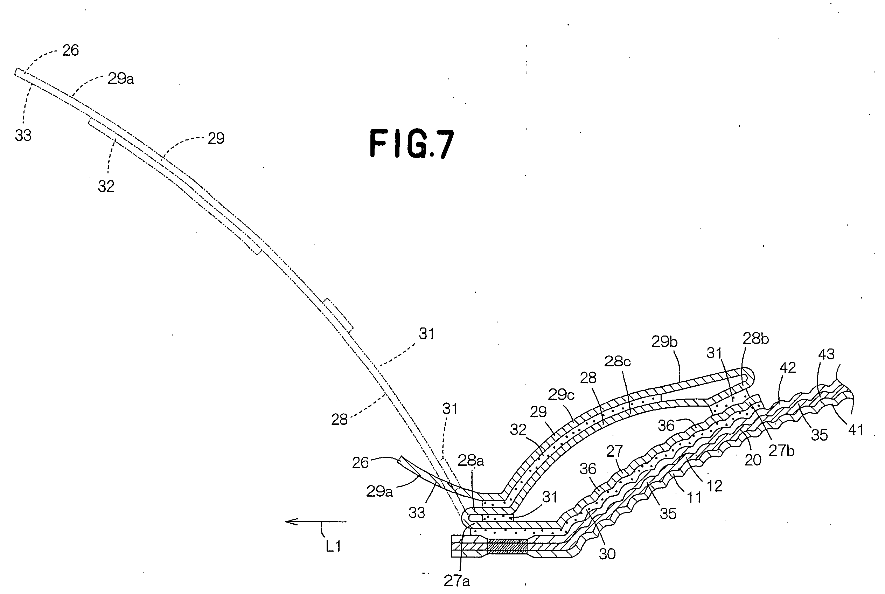

Fig. 6 is a perspective view showing a diaper 10C according to still another embodiment of the present invention and Fig. 7 is a sectional view taken along a line 7-7 in Fig. 6. In Fig. 6, the waist-circumferential direction is indicated by the arrow L and the longitudinal direction is indicated by the arrow M. In Fig. 7, the first and second free subsections 28, 29 of the tape tabs 26 are developed outward in the transverse direction of the diaper 10C as indicated by chain double-dashed lines.

The diaper 10C comprises the liquid-pervious topsheet 11 facing the wearer's skin, the liquid-impervious backsheet 12 facing away from the wearer' s skin and the liquid-absorbent core 13 interposed between the top- and backsheets 11, 12. The topsheet 11 is formed by hydrophilic fibrous nonwoven fabric and the backsheet 12 is formed by hydrophobic fibrous nonwoven fabric 41 and the backsheet 12 is formed by composite fibrous nonwoven fabric consisting of two layers of hydrophobic fibrous nonwoven fabric 42, 43 bonded to each other.

The diaper 10C is configured in the pants-type having the waist-hole 17 and a pair of leg-holes 18 and has the front and rear waist regions 14, 16 opposed to each other and the crotch region 15 extending between these waist regions 14, 16. The diaper 10C further comprises the longitudinally opposite end portions 19 extending in the transverse direction and the lateral portions 20, 21, 22 extending in the longitudinal direction.

Between the waist-opening 17 and a pair of the leg-holes 17, the lateral portions 20, 22 of the front and rear waist regions 14, 16 are bonded together by means of a plurality of heat-sealing lines 23 arranged intermittently in the longitudinal direction in the vicinity of the respective marginal edge zones of the lateral portions 20, 22. The opposite lateral portions 21 of the diaper 10C in the crotch region 15 describe circular arcs which are convex inward in the transverse direction so that the crotch region 15 has a width narrower than those of the front and rear waist regions 14, 16. Accordingly, the diaper 10C presents a substantially hourglass-like planar shape as the front and rear waist regions 14, 16 are disconnected from each other and the diaper 10C is developed in the longitudinal direction. Along the waist-surrounding end portions 19 and the lateral portions 20, 21, 22, the top- and backsheets 11, 12 are placed upon each other and have respective inner surfaces intermittently bonded together by means of adhesive (not shown).

The longitudinally opposite end portions 19 are provided with a plurality of waist-surrounding elastic members 24 extending in the transverse direction and the lateral portions 21 of the crotch region 15 are provided with a plurality of leg-surrounding elastic members 25 so that these elastic members 24, 25 may be contractible. Between the waist-surrounding elastic members 24 and the leg-surrounding elastic members 25, there are provided with a plurality of auxiliary elastic members 34 spaced one from another in the longitudinal direction and extending in the transverse direction so that these auxiliary elastic members 34 may be contractible. These auxiliary elastic members 34 are arranged substantially at regular intervals in the longitudinal direction.

The waist-surrounding elastic members 24 and the leg-surrounding elastic members 25 are interposed between the top- and backsheets 11, 12 and intermittently bonded to the respective inner surfaces of these sheets 11, 12 by means of adhesive (not shown). The portions of these auxiliary elastic members 34 interposed between the backsheet 12 and the core 13 are intermittently bonded to the inner surface of the backsheet 12 by means of adhesive (not shown) while the portions thereof interposed between the top- and backsheets 11, 12 are intermittently bonded to the respective inner surfaces of the top- and backsheets 11, 12 by means of adhesive (not shown). While the auxiliary elastic members 34 are illustrated as distributed in substantially upper halves of the front and rear waist regions 14, 16, it is possible to distribute these auxiliary elastic members 34 in substantially entire extents of the front and rear waist regions 14, 16, respectively.

In the front waist region 14, a pair of tape tabs 26 are attached to the respective lateral portions 20 and extend in the transverse direction. Each of these tape tabs 26 is folded so as to present a Z-shaped cross-section and adapted to be unfolded from the associated lateral portion 20 outward in the transverse direction as indicated by double chain-dashed line in Fig. 7. The tape tab 26 has the fixed section 27 extending in the transverse direction, the first free tape subsection 28 being contiguous to the fixed section 27 and extending also in the transverse direction and the second free subsection 29 being contiguous to the first free subsection 28 and extending in the transverse direction.

The fixed section 27 has its inner surface permanently bonded to the outer surface of the backsheet 12 by means of the adhesive 30. The fixed section 27 is bonded to the backsheet 12 while the auxiliary elastic members 34 are stretched at a predetermined ratio in the transverse direction. The first free subsection 28 is folded along an outer end 27a of the fixed section 27 toward the outer surface of the fixed section 27. The first free subsection 28 is placed upon the outer surface of the fixed section 27 and temporarily bonded to the outer surface of the fixed section 27 by means of pressure-sensitive adhesive 31 serving as fastening means applied on the inner surface of the first free subsection 28. The second free subsection 29 is folded along an inner end portion 28b of the first free subsection 28 toward the outer surface of the first free subsection 28. The second free subsection 29 is placed upon the outer surface of the first free subsection 28 and temporarily bonded to the outer surface of the first free subsection 28 by means of pressure-sensitive adhesive 32 serving as fastening means applied on the inner surface of the second free subsection 29. The second free tape subsection 22 is formed in the vicinity of its outer end portion 29a with a finger grip 33 coated with no pressure-sensitive adhesive.

The pressure-sensitive adhesive 31 is applied on the entire inner and outer end portions 28a, 28b of the first free subsection 28 and the pressure-sensitive adhesive 32 is applied on the intermediate portion 29c of the second free subsection 29 except the finger grip 33 and the inner end portion 29c of the second free subsection 29. On the other hand, the intermediate portion 28c of the first free subsection 28 is not coated with the pressure-sensitive adhesive 31 and the intermediate portion 29c is not temporarily bonded to the fixed section 27. The inner and outer end portions 27a, 27b of the fixed section 27 as well as the first subsection 28 are coated on the outer surfaces thereof with release agent (not shown).

Just like the case shown by Fig. 1, the tape tabs 26 are not only useful for disposal of the used diaper 10C contaminated with bodily discharges but also for adjustment of the waist-circumferential dimension N of the diaper 10C put on the wearer's body. Disposal of the diaper 10C contaminated with bodily discharges as well as the adjustment of the waist-circumferential dimension N of the diaper 10C put on the wearer's body may be carried out in accordance with the same procedure as has been described with respect to the embodiment shown by Fig. 1. The diaper 10C is maintained in the rolled up state and can be disposed in this rolled up state. The tape tabs 26 are useful not only for disposal of the used diaper 10C but also for adjustment of the waist-circumferential dimension N as the diaper 10A is put on the wearer's body. To adjust the waist-circumferential dimension N of the diaper 10C, the tape tabs 26 may be developed outward in the transverse dimension, then the tape tabs 26 may be pulled toward the transversely middle point and the second free subsections 29 of the tape tabs 26 may be anchored on the outer surface of the rear waist region 16 (the outer surface of the backsheet 12). In this way, the waist-circumferential dimension N of the diaper 10C can be reduced.

In the diaper 10C, a content of finishing textile oil in the fibrous nonwoven fabric 42 forming the backsheet 12 is 0.04wt% or less based on a weight of fiber constituting the nonwoven fabric 42 and an adhesive strength of the fixed section 27 of the tape tab 26 to the backsheet 12 is 4N/20mm or higher as measured after the diaper 10C has been left at a temperature of 50°C and at a humidity of 60% for a week. The content of finishing textile oil in nonwoven fabric 42 was measured in accordance with solvent extraction process specified by JIS L 1015 7.22. The adhesive strength of the fixed section 27 to the backsheet 12 was measured by the same method as has been described with respect to the embodiment shown by Fig. 1. Regarding the adhesive 30, 31, 32, the release agent and the finishing textile oil also, those as have been described in connection with the embodiment shown by Fig. 1 may be used.

If the content of the finishing textile oil for the nonwoven fabric 42 is less than 0.04wt% based on the weight of the component fibers constituting the nonwoven fabric 42, deterioration of the adhesive strength of the adhesive 30 can be avoided and the desired adhesive strength of the fixed section 27 of the tape tab 26 to the backsheet 12 can be maintained even after the diaper 10C has been left for a long period.

In the diaper 10C, the top- and backsheets 11, 12 of the front and rear waist regions 14, 16 are formed with a plurality of gathers 35 as the waist-surrounding elastic members 24 and the auxiliary elastic members 34 contract inward in the transverse direction. At the same time, contraction of these elastic members 24, 34 causes a dimension of the fixed section 27 as measured between its inner and outer ends 27a, 27b to be reduced and thereby causes the fixed section 27 to be entirely formed with a plurality of gathers 36. Of the tape tab 26, the intermediate portion 28c of the first free subsection 28 is free from the fixed section 27, so the respective intermediate portions 28c, 29c of the first and second free subsections 28, 29 curve outward so as to be spaced from the fixed section 27.

It is more anxious in the diaper 10C than the diaper 10A of Fig. 1 that the fixed section 27 might be unintentionally peeled off from the backsheet 12, because a plurality of gathers 36 are formed on the fixed section 27 of the tape tab 26 as the elastic members 24, 34 contract in the case of the diaper 10C while none of such gathers is formed on the fixed section 27 of the tape tab 26 in the case of the diaper 10A. However, the adhesive strength of the fixed section 27 to the backsheet 12 of 4N/20mm or less reliably prevents the fixed section 27 from being peeled off from the outer surface of the backsheet 12 even when the fixed section 27 is formed with a plurality of gathers 36.

The diaper 10C is primarily characterized in that the respective intermediate portions 28c, 29c of the first and second free subsections 28, 29 are curved substantially in circular arcs so as to be spaced from the fixed section 27. With such arrangement, the second free subsection 29 may be pulled outward in the transverse direction with the finger grip 33 held in the hand to generate an appropriate peel force between the inner and outer end portions 28a, 28b of the first free subsection, on one hand, and the inner and outer end portions 29a, 29b of the second free subsection 29, on the other hand. This peel force facilitates the second free subsection 29 to be peeled off from the outer surface of the first free subsection 28 and simultaneously facilitates the first free subsection 28 to be peeled off from the outer surface of the fixed section 27.

In this diaper 10C also, the content of the finishing textile oil in the fibrous nonwoven fabric 41 forming the topsheet 11 is preferably 0.04wt% or less based on the weight of the component fibers constituting the nonwoven fabric 41 and the adhesive strength between the top- and backsheets 11, 12 is preferably 0.15N/25mm or higher. The content of the finishing textile oil in the fibrous nonwoven fabric 43 forming the backsheet 12 also is preferably 0.04wt% or less based on the weight of the component fibers constituting the nonwoven fabric 43. The adhesive strength between the nonwoven fabric 42 and the nonwoven fabric 43 is preferably 0.15N/25mm or higher. The content of the finishing textile oil in the nonwoven fabric 43 is measured by the same method as for the nonwoven fabric 42. The adhesive strength between the nonwoven fabric 42 and the nonwoven fabric 43 top- and backsheets 11, 12 is measured by the same method as for the adhesive strength of the tape tab 26 to the backsheet 12 (It should be understood that the sheet sample for measurement comprises these two layers of nonwoven fabric 42, 43 bonded to each other).

If the content of the finishing textile oil in the fibrous nonwoven fabric 41 exceeds 0.04wt%, the finishing textile oil may progressively deteriorate the adhesive force of adhesive as time elapses until the adhesive strength between the nonwoven fabric layers 42, 43 may fall below 0.15N/20mm. Assumed that the adhesive strength between the nonwoven fabric layers 42, 43 is less than 0.15N/25mm, these nonwoven fabric layers 42, 43 may sometimes be peeled off from each other as the tape tabs 26 are pulled in the transverse direction.

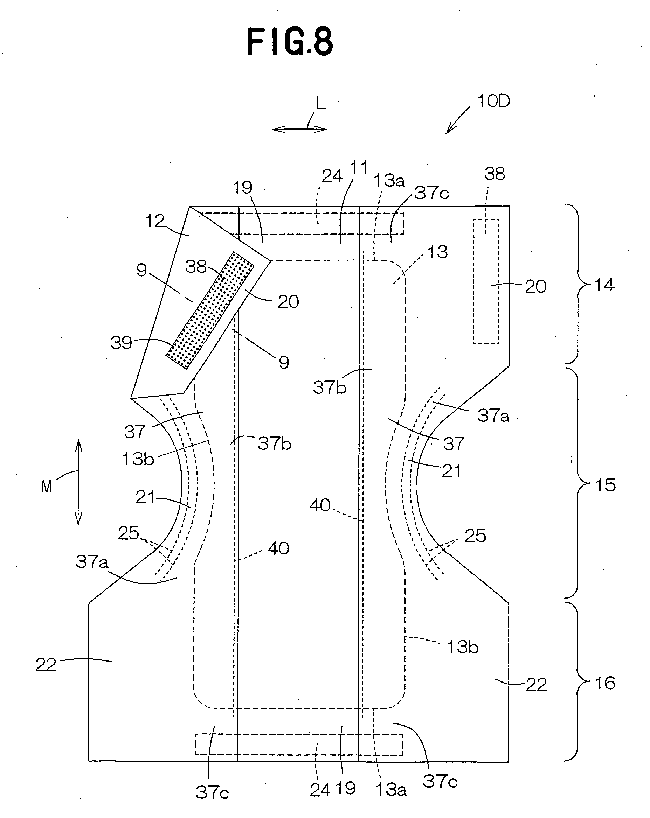

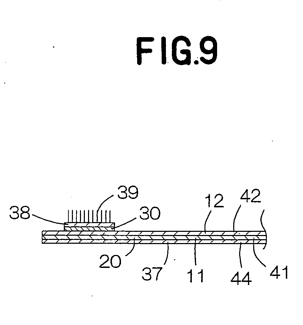

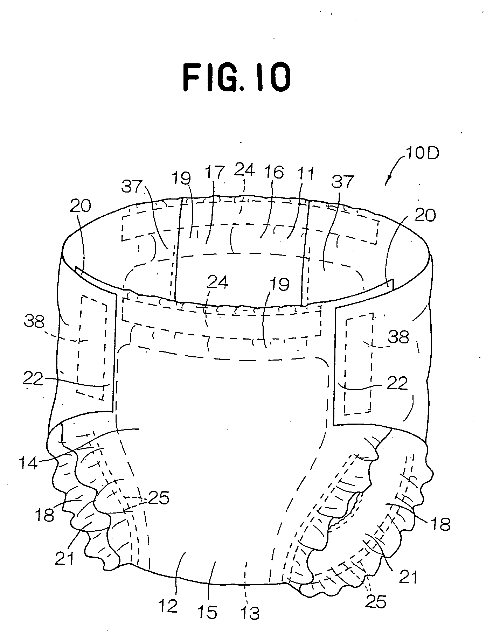

Fig. 8 is a plan view showing a diaper 10D according to further another embodiment of the present invention, Fig. 9 is a sectional view taken along a line 9-9 in Fig. 8 and Fig. 10 is a perspective view showing the diaper 10D with the front and rear waist regions 14, 16 connected to each other along the lateral portions 20, 22 thereof. In Fig. 8, the transverse direction is indicated by the arrow L, the longitudinal direction is indicated by the arrow M and one of the lateral portions 20 in the front waist region 14 is shown as folded back onto the outer surface of the topsheet 11.

The diaper 10D comprises the liquid-pervious topsheet 11, the liquid-impervious backsheet 12 and the liquid-absorbent core 13 interposed between the top- and backsheets 11, 12 and bonded to the inner surface of at least one of these sheets 11, 12.

The diaper 10D is composed of, in the longitudinal direction, the front waist region 14, the rear waist region 16 and the crotch region 15 extending between these waist regions 14, 16. The topsheet 11 is formed by hydrophilic fibrous nonwoven fabric 41 and the backsheet 12 is formed by hydrophobic fibrous nonwoven fabric 42.

The diaper 10D is substantially contoured by the longitudinally opposite end portions 19 extending in the transverse direction and the transversely opposite lateral portions 20, 21, 22 extending in the longitudinal direction. In the crotch region 15, the lateral portions 21 describe circular arcs which are convex inward in the transverse direction of the diaper 10D so that the diaper 10D presents a substantially hourglass-like planar shape. Along the end portions 19 and the lateral portions 20, 21, 22, the top- and backsheets 11, 12 are put flat together and have respective inner surfaces intermittently bonded together by means of adhesive (not shown).

The waist-surrounding end portions 19 are provided with a plurality of waist-surrounding elastic members 24 extending in the transverse direction so that these elastic members 24 may be contractible. The lateral portions 21 of the crotch region 15 are provided with a plurality of leg-surrounding elastic members 25 so that these elastic members 25 may be contractible. These elastic members 24, 25 are interposed between the top- and backsheets 11, 12 and intermittently bonded to the inner surfaces of these sheets 11, 12 by means of adhesive (not shown).

The lateral portions 20, 21, 22 are provided with liquid-resistant leak-proof sheets 37 attached thereto and extending in the longitudinal direction. Each of the leak-proof sheet 37 is formed by hydrophobic fibrous nonwoven fabric 44. The leak-proof sheets 37 have fixed side portions 37a extending in the longitudinal direction in parallel to the lateral portions 20, 21, 22, respectively, movable portions 37b extending in the longitudinal direction in parallel to these fixed portions 37b and fixed end portions 37c defined by longitudinally opposite end portions of the respective movable portions 37c. The respective movable portions 37b are provided along inner side edges thereof with stretchable elastic members 40 bonded in stretched state to the respective inner side edges. The elastic members 40 are firmly bonded to the respective movable portions 37b and covered with parts of these movable portions 37b. The longitudinally opposite fixed end portions 37c are collapsed inward in the transverse direction of the diaper 10D and firmly bonded to the outer surface of the topsheet 11 in this collapsed state. The movable portions 37b rise on the topsheet 11 and form barriers against bodily discharges as the diaper 10D longitudinally curves with the topsheet inside and correspondingly the elastic members 40 contract.

In the front waist region 14, the transversely opposite lateral portions 20 are provided with a pair of tape tabs 38 extending in the longitudinal direction. These tape tabs 38 are used to connect the lateral portions 20 of the front waist region 14 and the lateral portions 22 of the rear waist region 16 to each other. Each of the tape tabs 38 has a rectangular shape which is longer than is wider and has its inner surface bonded to the outer surface of the backsheet 12 by means of adhesive 30. The tape tab 38 is formed on its entire outer surface with a plurality of hooks 39 constituting so-called mechanical fastener.

The diaper 10D may be put on the wearer' s body in a manner as follows: The inner surfaces of the lateral portions 22 of the rear waist region 16 are put flat together the outer surface of the lateral portions 20 of the front waist region 14 and the lateral portions 22 are pressed against the lateral portions 20. Thereupon the hooks 39 formed on the tape tab 38 are entangled with the component fibers of the nonwoven fabric constituting the backsheet 12 and thereby the tape tab 38 comes in engagement with the inner surface of the lateral portions 22 of the rear waist region 16. In this way, the lateral portions 20, 22 of these front and rear waist regions 14, 16 are connected to each other. As shown by Fig. 10, the diaper 10D is formed with the waist-hole 17 and a pair of leg-holes 18 as the front and rear waist regions 14, 16 are connected to each other. Having connected the front and rear waist regions 14, 16 together, legs of the wearer are guided through the waist-hole 17 into the respective leg-holes 18 and then the diaper 10D is slid upward along the wearer's waist.

In the diaper 10D, a content of finishing textile oil (not shown) in the fibrous nonwoven fabric 42 forming the backsheet 12 is 0.04wt% or less based on a weight of fiber constituting the nonwoven fabric 42 and an adhesive strength of the fixed section 27 of the tape tab 26 to the backsheet 12 is 4N/20mm or higher as measured after the diaper 10A has been left at a temperature of 50°C and at a humidity of 60% for a week. The content of finishing textile oil in nonwoven fabric 42 was measured in accordance with solvent extraction process specified by JIS L 1015 7.22. The adhesive strength of the tape tab 38 to the backsheet 12 was measured by the method same as the method having been described in connection with Fig. 1. The adhesive 30 and the finishing textile oil are same as those used in the embodiment shown by Fig. 1.

If the content of the finishing textile oil for the nonwoven fabric 42 is less than 0.04wt% based on the weight of the component fibers constituting the nonwoven fabric 42, deterioration of the adhesive strength of the adhesive 30 can be avoided and the desired adhesive strength of the fixed section 27 of the tape tab 26 to the backsheet 12 can be maintained even after the diaper 10D has been left for a long period. The tape tab 38 can be held on the backsheet 12 at the adhesive strength of 4N/20mm or higher even after the diaper 10D has been left at a temperature of 50°C and a humidity of 60% for a week, so there is no apprehension that the tape tab 26 might be unintentionally peeled off from the outer surface of the backsheet 12 even if engagement and disengagement between the tape tabs 38 and the lateral portions 22 of the rear waist region 16 are repeated after the diaper 10D has been left for a long period.

In this diaper 10D also, the content of the finishing textile oil in the fibrous nonwoven fabric 41 forming the topsheet 11 also is preferably 0.04wt% or less based on the weight of the component fibers constituting the nonwoven fabric 41 and the adhesive strength between the top- and backsheets 11, 12 is preferably 0.15N/25mm or higher.

Stock material for the topsheet 11 may be selected from a group consisting of hydrophilic fibrous nonwoven fabric, hydrophobic fibrous nonwoven fabric having a plurality of apertures and finely perforated plastic film. The fibrous nonwoven fabric 41, 44 forming the topsheet 11 and the leak-proof sheet 37 may be selected from a group consisting of spun lace nonwoven fabric, needle punch nonwoven fabric, melt blown nonwoven fabric, thermal bond nonwoven fabric, spun bond nonwoven fabric and chemical bond nonwoven fabric.

While the nonwoven fabric 42, 32 forming the backsheet 12 may be preferably selected from a group consisting of spun bond nonwoven fabric and spun lace nonwoven fabric, it is possible to use needle punch nonwoven fabric, melt blown nonwoven fabric, thermal bond nonwoven fabric or chemical bond nonwoven fabric. It is also possible to use, as stock material for the backsheet 12 and the leak-proof sheet 37, composite nonwoven fabric comprising melt blown nonwoven fabric and spun bond nonwoven fabric laminated on one or both surfaces of the melt blown nonwoven fabric (i.e., SM-nonwoven fabric, SMS-nonwoven fabric or SMMS-nonwoven fabric). When the composite nonwoven fabric consisting of spun bond nonwoven fabric and melt blown nonwoven fabric is used as stock material for the backsheet 12, the tape tabs 26, 38 are preferably bonded to the outer surface of the spun bond nonwoven fabric.

Composite fiber of the nonwoven fabric 41, 42, 43, 44 may be selected from a group consisting of polyester-, polyacrylonitrile-, polyvinyl chloride-, polyethylene- and polystyrene- fibers. These component fiber may be sheath-core-type conjugated fiber, side-by-side-type conjugated fiber, modified hollow fiber, microporous fiber or bond-type conjugated fiber.

The core 13 is a mixture of fluff pulp and super-absorbent polymer grains or a mixture of fluff pulp, super-absorbent polymer grains and thermoplastic synthetic resin fiber, in both cases, compressed to a predetermined thickness. Preferably, the core 13 is entirely covered with a liquid-pervious sheet such as tissue paper or hydrophilic fibrous nonwoven fabric in order to prevent the core 13 from getting out of shape and at the same time in order to prevent the polymer grains from falling off. The polymer grains may be selected from a group consisting of starch-based polymer grains, cellulose-based polymer grains or synthetic polymer grains.

Stock material for the tape tabs 26, 38 may be selected from a group consisting of flexible plastic film made of thermoplastic synthetic resin and fibrous nonwoven fabric. It is also possible to use a composite sheet of plastic film and fibrous nonwoven fabric laminated on each other as stock material for these tape tabs 26, 38. Preferably, spun bond nonwoven fabric or spun lace nonwoven fabric is used as the fibrous nonwoven fabric forming the tape tabs 26, 38.

Adhesive is preferably applied on the topsheet 11, the backsheet 12 and the leak-proof sheets 37 in a pattern selected from a group consisting of spiral pattern, wave-pattern, zigzag pattern, dot-pattern and striped patter. Application of the adhesive on these sheets 11, 12, 37 in such patterns ensures that these sheets 11, 12, 37 are intermittently bonded one to another, the core 13 is intermittently bonded to the sheets 11, 12 and the elastic members 24, 25, 40 are intermittently bonded to the sheets 11, 12, 37. Bonding of the sheets 11, 12, 37 one to another, bonding of the core 13 to the sheets 11, 12 and bonding of the elastic members 24, 25, 40 to the sheets 11, 12, 37 may be achieved by, in addition to use of adhesive, using the welding technique such as heat-sealing or sonic sealing.

While each of the tape tabs 26 used by the diapers 10A, 10B, 10C shown by Figs. 1, 4 and 6, respectively, is a single tape tab folded so as to present a Z-shaped cross-section, it is possible to form the fixed section 27 and the first and second free subsections 28, 29 by separate tape tabs which are then connected one to another and folded in the Z-shape. If the tape tabs 26 are formed by fibrous nonwoven fabric in the diapers 10A, 10B, 10C, the adhesive 31, 32 may be replaced by the hooks constituting so-called mechanical fastener formed on the inner surfaces of the first and second free subsections 28, 29. In the diapers 10A, 10B, 10C shown by Figs. 1, 4 and 6, it is also possible to attach the tape tabs 26 to the opposite lateral portions 22 of the rear waist region 16 and to bond the fixed sections 27 of the tape tabs 26 to the outer surface of the backsheet 12 in the rear waist region 16 by means of the adhesive 30. Furthermore, it is possible in these diapers 10A, 10B, 10C to provide a single tape tab 26 extending in the longitudinal direction on the transversely middle zone of the front or rear waist region 14, 16 and to bond the fixed section 27 of this tape tab 26 to the outer surface of the backsheet 12 by means of the adhesive 30.

In the diaper 10C shown by Fig. 6, it is possible to form the top- and backsheets 11, 12, at least the backsheet 12 by elastically stretchable fibrous nonwoven fabric. In this case, it is unnecessary to attach the auxiliary elastic members 34 to the backsheet 12. If the backsheet 12 of the diaper 10C is formed by such elastically stretchable fibrous nonwoven fabric, the fixed sections 27 of the respective tape tabs 26 should be bonded to the backsheet 12 while the backsheet 12 are being stretched in the transverse direction. It is possible to use, as the stretchable fibrous nonwoven fabric, spun bond nonwoven fabric or melt blown nonwoven fabric formed by stretchable fiber obtained by melt spinning thermoplastic elastomer resin fiber. It is also possible to use composite nonwoven fabric comprising fibrous nonwoven fabric of crimped fiber obtained by melt spinning thermoplastic synthetic resin such as polypropylene, polyethylene or polyester laminated on one or both surfaces of stretchable fibrous nonwoven fabric formed by thermoplastic elastomer resin fiber. In the diaper 10D shown by Fig. 8, it is also possible to provide the tape tabs 38 on the opposite lateral portions 22 of the rear waist region 16 and to bond the inner surface of the tape tabs 38 to the outer surface of the backsheet 12 in the rear waist region 16.

The disposable wearing article according to the present invention is primarily characterized in that the content of the finishing textile oil in the nonwoven fabric is 0.04wt% or less based on the weight of the component fibers constituting the nonwoven fabric. This feature reliably prevents the finishing textile oil from deteriorating the adhesive force of the adhesive and the desired adhesive strength of the adhesive can be avoided and the desired adhesive strength of the tape tabs to the backsheet can be maintained even after the article has been left for a long period. The tape tabs can be held on the backsheet at the adhesive strength of 4N/20mm or higher even after the article has been left for a long period, so there is no apprehension that the tape tabs might be unintentionally peeled off from the outer surface of the backsheet even when the tape tabs are pulled in the transverse direction or repeatedly engaged with and disengaged from the backsheet.

In the wearing article in which each of the tape tabs comprises the fixed section and the free section, the tape tabs can be used to hold the article contaminated with bodily discharges in a predetermined shape for disposal and can be also to adjust a waist-circumferential dimension put on the particular wearer.

In the wearing article in which any one of the front and rear waist regions is provided on the opposite lateral portions of this waist region with the tape tabs comprising the fastening means, these tape tabs may be used to connect the respective lateral portions of the front and rear waist regions.

## Claims

1. A disposable wearing article having front and rear waist regions opposed to each other, a crotch region extending between these waist regions, and tape tabs attached to the outer surface of the backsheet facing away from a wearer's skin, said disposable wearing article further comprising:
said backsheet being formed by a fibrous nonwoven fabric and a content of finishing textile oil in said nonwoven fabric is 0.04wt% or less (as measured in accordance with the solvent extraction process specified by JIS L 1015 7.22) on the basis of a weight of fibers constituting said nonwoven fabric and said tape tabs being permanently bonded to the outer surface of said backsheet by means of adhesive.

2. The wearing article according to Claim 1, wherein each of said tape tabs comprises a fixed section permanently bonded to the outer surface of said backsheet by means of said adhesive and a first free subsection being contiguous to said fixed section so as to be placed upon the outer surface of said fixed section and temporarily bonded to the outer surface of said fixed section by fastening means formed on the inner surface of said first free subsection.

3. The wearing article according to Claim 2, wherein said adhesive applied on said fixed section of said tape tab is pressure-sensitive adhesive.

4. The wearing article according to Claim 3, wherein each of said tape tabs further comprises a second free subsection provided on transversely opposite side edge portions of said front or rear waist region so as to extend laterally outward therefrom, respectively, and being contiguous to said first free subsection so as to be placed upon the outer surface of said first free subsection and wherein said second free subsection is temporarily bonded to the outer surface of said first free subsection by fastening means formed on the inner surface of said second free subsection.

5. The wearing article according to Claim 1, wherein said tape tabs are provided on transversely opposite side edge portions of said front or rear waist region so as to extend longitudinally and means to connect said front and rear waist regions to each other are formed on the outer surfaces of said tape tabs.

6. The wearing article according to any one of Claims 1 through 5, wherein adhesive strength of said fixed tape section to said backsheet is 4N/20mm or higher as measured after said wearing article has been left at a temperature of 50°C and at a humidity of 60% for a week.

7. The wearing article according to any one of Claims 1 through 5, wherein said backsheet has elastic stretchability in its transverse direction and said tape tabs are bonded to said backsheet while said backsheet is stretched in its transverse direction.

8. The wearing article according to Claim 6, wherein a plurality of elastic members spaced one from another by a given dimension are contractibly attached to said backsheet and said elastic stretchability of said backsheet in its transverse direction is given by said elastic members.

9. The wearing article according to any one of Claims 1 through 7, wherein said fibrous nonwoven fabric constituting said backsheet is made of spun bond nonwoven fabric or spun lace nonwoven fabric.

10. The wearing article according to any one of Claims 2 through 8, wherein said fastening means are provided in the form of pressure-sensitive adhesive or hooks constituting so-called mechanical fastener.
